# EUROPÄISCHE PATENTANMELDUNG

(11) **EP 1 417 949 A1**
(43) Veröffentlichungstag der Anmeldung: **12.05.2004**
(21) Anmeldenummer: 03022367.1
(22) Anmeldetag: 06.10.2003
(51) Int. Cl.: A61H 39/00, A61N 1/16, C02F 1/00

(54) **Einrichtung zur Behandlung eines menschlichen, tierischen oder sonstigen, Flüssigkeit enthaltenden Körpers, einer Flüssigkeit, insbesondere Wasser, oder eines Raumes**

(30) Priorität: 12.10.2002 DE 10247685; 07.12.2002 DE 20218990 U
(71) Anmelder: Stoll, Hermann, 61197 Florstadt (DE)
(72) Erfinder: Stoll, Hermann, 61197 Florstadt (DE)
(74) Vertreter: Körner, Volkmar Horst

(57) **Zusammenfassung**

Eine Einrichtung zur Behandlung eines menschlichen oder tierischen Körpers hat untereinander verbundene Platten (1, 3) mit einer vorgesehenen Polung und mit einer Kontaktseite (2, 5) zum Kontaktieren mit dem Körper. Die Platten (1, 3) lassen sich beispielsweise anstelle von Akupunkturnadeln einsetzen.

## Beschreibung

Die Erfindung betrifft eine Einrichtung zur Behandlung eines menschlichen, tierischen oder sonstigen, Flüssigkeit enthaltenden Körpers, einer Flüssigkeit, insbesondere Wasser, oder des Raumes.

Es ist allgemein bekannt, den menschlichen oder tierischen Körper mittels Akupunktur zu behandeln. Hierbei werden spezielle Akupunkturnadeln in bestimmte, der vorgesehenen Behandlung entsprechende Stellen der Haut gestochen. Nachteilig bei der bekannten Akupunktur ist jedoch, dass sie schmerzhaft ist und nur von besonders erfahrenen Praktikern angewandt werden kann, da die bestimmten Stellen des Körpers genau getroffen werden müssen.

Weiterhin sind Einrichtungen zur Behandlung von Körpern und Flüssigkeiten, insbesondere Wasser bekannt geworden, welche Sensoren und eine Energiezufuhr aufweisen, mit denen die Energie des Körpers oder der Flüssigkeit auf zumindest den vorgesehenen Wert von 6500 der Bovismetrie Skala aufgeladen werden kann. Eine solche Einrichtung ist jedoch sehr aufwändig.

Weiterhin sind Einrichtungen zum Schutz vor Strahlungen aus der Umgebung, beispielsweise aus Richtung des Erdbodens bekannt geworden, mit denen Räume vor den Strahlungen geschützt werden können. Die bekannten Einrichtungen erfordern jedoch einen sehr großen Montageaufwand.

Der Erfindung liegt das Problem zugrunde, eine Einrichtung der eingangs genannten Art so zu gestalten, dass sie besonders einfach herzustellen und anzuwenden ist.

Dieses Problem wird erfindungsgemäß dadurch gelöst, dass zwei Platten über zumindest eine Leitung miteinander verbunden sind, dass die Platten eine unterschiedliche Polarität aufweisen und dass zumindest eine der Platten eine Kontaktseite zum in Kontakt bringen mit dem Körper hat.

Dank der Erfindung lassen sich die Platten auf vorgesehene Stellen des menschlichen oder tierischen Körpers oder einem Gefäß mit der Flüssigkeit auflegen bzw. die Platten mit den Stellen in Kontakt bringen. Da Platten größere Abmessungen aufweisen als Spitzen von Akupunkturnadeln, ist eine exakte Positionierung der Platten auf die vorgesehenen Bereiche des menschlichen oder tierischen Körpers nur von untergeordneter Bedeutung. Deshalb kann im einfachsten Falle der Patient die Behandlung nach Anleitung selbst vornehmen. Da die erfindungsgemäße Einrichtung keinen elektrischen Strom und damit auch keine Anschlusskontakte benötigt, lässt sie sich besonders kostengünstig herstellen. Die vorgesehene Polung und Energie lässt sich mittels eines Tensors oder Pendels feststellen und durch eine geeignete Wahl des Materials, der Größe der Platte und des in Kontakt bringens mit dem Körper einstellen und über die Bovismetrieskala kontrollieren und feststellen. Damit lässt sich für jede Person die für die Behandlung oder für die vorgesehene Flüssigkeit erforderliche Energie der Platten festlegen. Bei dem sogenannten Yin- und Yan-Ausgleich wird durch Auflage der Platten auf die Meridiane das innere Gleichgewicht des Menschen oder des Tieres hergestellt. Auffüllungen der Chakren sind ebenfalls möglich. Weiterhin kann mit der sehr einfach aufgebauten erfindungsgemäßen Einrichtung beispielsweise ein Wohnraum oder ein Haus vor Strahlen aller Art, beispielsweise vor Erdstrahlen geschützt werden. Die miteinander verbundenen Platten können dabei eine Kantenlänge von 30 mm oder den zu schützenden Räumen entsprechende und damit sehr große Abmessungen und Formen aufweisen.

Bei der Behandlung des menschlichen oder tierischen Körpers, der Flüssigkeit oder des Raumes besteht wahlweise die Möglichkeit, dass beide Platten mit den vorgesehenen Stellen in Kontakt gebracht werden oder dass nur die Platte mit der vorgesehenen Polarität mit der vorgesehenen Stelle in Kontakt gebracht wird und die zweite Platte auf den Erdboden gelegt wird. Wesentlich für die Erfindung ist, dass die Platten Energie zuzuführen oder abzuziehen vermögen und diese damit an ihrer Kontaktseite pluspolig oder minuspolig sind und ihre Energie an den Körper, die Flüssigkeit oder den Raum wieder abgeben können. Alternativ dazu kann der Raum auch dadurch geschützt werden, dass die Strahlen von den Platten absorbiert oder reflektiert werden. Deshalb ist während der Behandlung des Körpers, der Flüssigkeit oder des Raumes keine elektrische Energiezufuhr erforderlich. Im Sinne der Erfindung ist pluspolig und minuspolig mit den ebenfalls gebräuchlichen Ausdrücken rechtsdrehend und linksdrehend gleichbedeutend.

Die Platten sind vorzugsweise rechteckig, quadratisch oder rund mit einer Kantenlänge oder einem Radius von 35 bis 300 mm oder größer gestaltet.

Eine Abstrahlung von die nähere Umgebung beeinflussender Energie von elektrischen oder elektronischen Einrichtungen, wie beispielsweise eines Mobilfunkmastes, eines Computers oder eines Stromkastens, lässt sich gemäß einer anderen vorteilhaften Weiterbildung der Erfindung einfach begrenzen, wenn bei einer Verbindung in einer Draufsicht rechteckiger ebener Platten mit zwei Leitungen die Leitungen an einander benachbarten Randseiten der Platten angeschlossen sind. Hierbei ist zumindest eine der Platten unmittelbar an dem Stromkasten oder dem Mobilfunkmast anzubringen und die andere Platte beispielsweise auf den Boden zu legen. Durch diese Anordnung lässt sich zudem im umgekehrten Fall ein Raum in einer Immobilie, beispielsweise einem Wohnhaus, oder in einem Kraftfahrzeug vor störender Energieeinstrahlung schützen, wenn die Platten wie beschrieben voneinander entfernt an der Immobilie oder dem Kraftfahrzeug angeordnet werden. Die Verminderung der Abstrahlung der Energie von den elektrischen oder elektronischen Einrichtungen ist prinzipiell auch mit einer einzigen Leitung zur Verbindung der Platten möglich, jedoch haben Versuche gezeigt, dass die Verbindung der Platten mit zwei Leitungen an einander benachbarten Randseiten eine Umpolung von linkspolig auf rechtspolig ermöglicht.

Eine vorgesehene Energetisierung von Wasser in einer Wasserleitung lässt sich gemäß einer anderen vorteilhaften Weiterbildung der Erfindung besonders einfach erreichen, wenn zwei Leitungen zur Kontaktierung einer gekrümmten, ein Rohr abdeckenden Platte an den Stirnseiten der Platte angeschlossen sind. Hierdurch ist die Form einer der Platten der Wasserleitung entsprechend angepasst. Die andere Platte kann auf den Boden von der Wasserleitung entfernt gelegt werden.

Bei ebenen quadratischen Platten werden die Leitungen vorzugsweise mittig an den Randseiten angeschlossen, wobei die minuspolige Platte auf den Erdboden gelegt wird. In einer Wasserleitung fließendes Wasser lässt sich gemäß einer vorteilhaften Weiterbildung der Erfindung pluspolig energetisieren, wenn die Leitungen außerhalb der Mitte der Stirnseiten der gekrümmten Platte angeschlossen sind, wobei eine der Leitungen an einer vorgesehenen Anströmseite der Platte links und die andere der Leitungen an einer Abströmseite rechts angeschlossen ist.

Die Erfindung lässt zahlreiche Ausführungsformen zu. Zur weiteren Verdeutlichung ihres Grundprinzips ist eine davon in der Zeichnung dargestellt und wird nachfolgend beschrieben. Diese zeigt in
- Fig. 1: eine erfindungsgemäße Einrichtung mit einer einzigen Leitung,
- Fig. 2: eine erfindungsgemäße Einrichtung mit zwei Leitungen
- Fig. 3: eine erfindungsgemäße Einrichtung mit einer gekrümmten Platte.

Die Einrichtung hat zwei rechteckige Platten 1, 3, von denen zumindest eine zum Kontakt mit einem menschlichen Körper, der Flüssigkeit oder des Raumes vorgesehen ist. Die Platten 1, 3 weisen hier jeweils eine Kontaktseite 2, 5, beispielsweise für den Kontakt mit dem Körper, der Flüssigkeit oder des Raumes auf. Die Platten 1, 3 sind über eine beispielsweise aus Kupfer gefertigte Leitung 4 miteinander verbunden. Hierfür weisen die Platten Bohrungen auf, in die Steckkontakte 6, 7 der Leitungen eingesteckt sind. Die Steckkontakte 6, 7 sind hier als handelsübliche Bananenstecker ausgebildet. Eine der Platten 1 ist an ihrer Kontaktseite 2 minuspolig und die andere Platte 3 ist an ihrer Kontaktseite 5 pluspolig. Selbstverständlich können die Platten auch an beiden Seiten Kontaktseiten haben. Mittels eines Tensors oder Pendels lässt sich die Polung und die Aufladung der Platten 1,3 mit der sogenannten Bovisenergie bestimmen, oder die Polung und die Aufladung sind an einer Bovismetrieskala ablesbar.

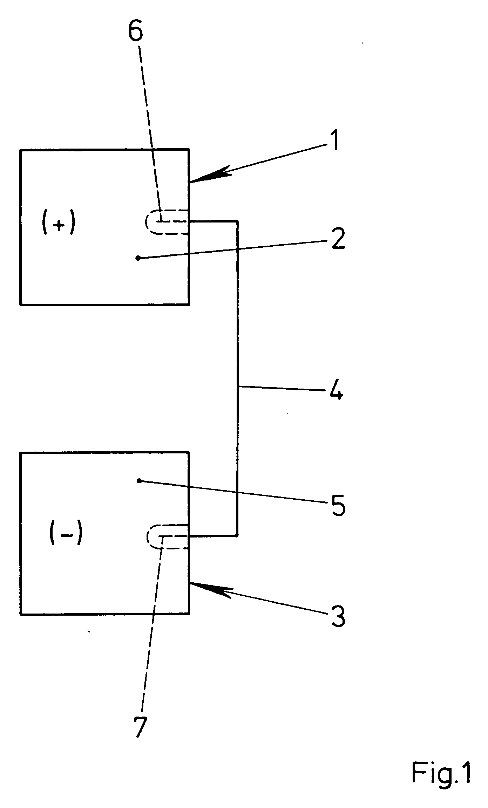

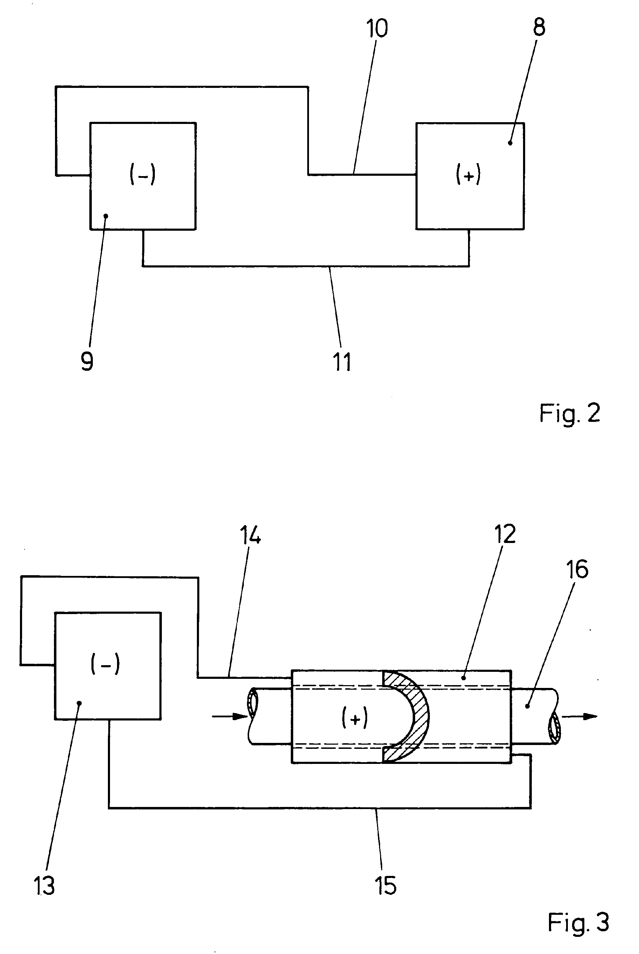

Figur 2 zeigt eine Einrichtung, welche sich von der aus Figur 1 vor allem dadurch unterscheidet, dass zwei quadratische Platten 8, 9 über zwei Leitungen 10, 11 miteinander verbunden sind. Die Leitungen 10, 11 sind mittig an aneinander angrenzenden Randseiten der Platten 8, 9 angeschlossen. Die Platten 8, 9 sind wie zu Figur 1 beschrieben aufgebaut.

Figur 3 zeigt eine Einrichtung mit einer an ein Rohr 16 angepassten gekrümmten Platte 12 und einer quadratischen Platte 13. Zur Verdeutlichung ist der Querschnitt der gekrümmten Platte 12 in der Zeichnung schraffiert dargestellt. Die quadratische Platte 13 ist wie in Figur 2 beschrieben mit zwei Leitungen 14, 15 verbunden. Die Leitungen 14, 15 sind außermittig an den Stirnseiten der gekrümmten Platte 12 angeschlossen. Diese Einrichtung dient zum Energetisieren von durch ein Rohr 16 strömendes Wasser. Zur Verdeutlichung ist die Strömung des Wassers mit Pfeilen gekennzeichnet. Eine der Leitungen 14 ist in der Ausströmrichtung des Wassers liegenden Stirnseite links an der gekrümmten Platte 12 angeschlossen, während die andere Leitung in der Abströmrichtung des Wassers liegenden Stirnseite rechts angeschlossen ist. Ansonsten sind die Platten 12, 13 wie zu Figur 1 beschrieben aufgebaut.

### Bezugszeichenliste

- 1: Platte
- 2: Kontaktseite
- 3: Platte
- 4: Leitung
- 5: Kontaktseite
- 6, 7: Steckkontakt
- 8, 9: Platte
- 10, 11: Leitung
- 12: Platte
- 13: Platte
- 14, 15: Leitung
- 16: Rohr

## Patentansprüche

1. Einrichtung zur Behandlung eines menschlichen, tierischen oder sonstigen, Flüssigkeit enthaltenden Körpers, einer Flüssigkeit, insbesondere Wasser, oder eines Raumes, **dadurch gekennzeichnet, dass** zwei Platten (1, 3, 8, 9, 12, 13) über zumindest eine Leitung (4, 10, 11, 14, 15) miteinander verbunden sind, dass die Platten (1, 3, 8, 9, 12, 13) eine unterschiedliche Polarität aufweisen und dass zumindest eine der Platten (1, 3, 8, 9, 12, 13) eine Kontaktseite (2, 5) zum in Kontakt bringen mit dem Körper, der Flüssigkeit oder des Raumes hat.

2. Einrichtung nach Anspruch 1, **dadurch gekennzeichnet, dass** die Platten (1, 3, 8, 9, 12, 13) Aluminium, Magnesium Mangan, Blei oder Kupfer enthalten oder im Wesentlichen aus diesen Materialien gefertigt sind.

3. Einrichtung nach Anspruch 1 oder 2, **dadurch gekennzeichnet, dass** die Leitung (4, 10, 11, 14, 15) Steckkontakte (6, 7) zur Verbindung mit den Platten (1, 3, 8, 9, 12, 13) aufweist.

4. Einrichtung nach zumindest einem der vorhergehenden Ansprüche, **dadurch gekennzeichnet, dass** die Platten (1, 3, 8, 9, 12, 13) rechteckig, quadratisch oder rund mit einer Kantenlänge oder einem Radius von 50 bis 300 mm gestaltet sind.

5. Einrichtung nach zumindest einem der vorhergehenden Ansprüche, **dadurch gekennzeichnet, dass** bei einer Verbindung in einer Draufsicht rechteckiger ebener Platten (8, 9, 12, 13) mit zwei Leitungen (10, 11, 14, 15) die Leitungen (10, 11, 14, 15) an einander benachbarter Randseiten der Platten (8, 9, 12, 13) angeschlossen sind.

6. Einrichtung nach zumindest einem der vorhergehenden Ansprüche, **dadurch gekennzeichnet, dass** zwei Leitungen (14, 15) zur Kontaktierung einer gekrümmten, ein Rohr abdeckenden Platte (12) an den Stirnseiten der Platte (12) angeschlossen sind.

7. Einrichtung nach zumindest einem der vorhergehenden Ansprüche, **dadurch gekennzeichnet, dass** die Leitungen (14, 15) außerhalb der Mitte der Stirnseite der gekrümmten Platte (12) angeschlossen sind, wobei eine der Leitungen (14) an einer vorgesehenen Anströmseite der Platte (12) links und die andere der Leitungen (15) an einer Abströmseite rechts angeschlossen ist.
